Europäisches Patentamt

(19) European Patent Office

Office européen des brevets

(11) Publication number:  **0 107 258**
**A1**

(12) # EUROPEAN PATENT APPLICATION

(21) Application number: **83201511.9**

(22) Date of filing: **21.10.83**

(51) Int. Cl.³: **A 61 N 1/14, A 61 N 1/22**

(30) Priority: **26.10.82 NL 8204117**

(43) Date of publication of application: **02.05.84**
**Bulletin 84/18**

(84) Designated Contracting States: **AT BE CH DE FR IT LI**
**LU NL SE**

(71) Applicant: **Hepag Health Education Products Ltd.,**
**Seebahnstrasse 31, Zürich (CH)**

(72) Inventor: **Van Baarle, Johannes Gerardus Antonius,**
**Willemsparkweg 194, NL-1071 HW Amsterdam (NL)**

(74) Representative: **de Boer, Hindrik Geert Jan et al, de**
**Lairessestraat 131-135, NL-1075 HJ Amsterdam (NL)**

(54) **Process for the manufacture of a garment, and garment made according to this process.**

(57) The invention relates to a garment provided with pressure electrodes interconnected by means of insulated conductive wires in order to equalize the electric skin potential between various acupuncture points on the human body. The invention also relates to textile materials from which the garment may be manufactured.

ACTORUM AG

60.90.538

## PROCESS FOR THE MANUFACTURE OF A GARMENT, AND GARMENT MADE ACCORDING TO THIS PROCESS

The invention relates to a process for the manufacture of a garment. Garments are generally made from flexible materials with a view to fitting close to the human body or tightly enclosing the human body or a part therof having a certain size. The material may be knitted wool, woven elastic and, in modern times especially, any material in a large category publicized as stretch materials.

It is an object of the present invention to utilize the mechanical properties of such garments in combination with the electrodes interconnected by these, so that in the wearing of such garments a constant and automatically maintained galvanic low-impedance contact between distant spots on the human skin is realized, without requiring any special provisions that would be found cumbersome. In the present invention this object is attained by the characteristic that on the side of the garment to be turned to the body at least a first and a second pressure electrode are fitted, which are in galvanic contact through an otherwise insulated flexible conductor of sufficient length to bridge the distance between the interconnected pressure electrodes as measured on the garment when applied to the body.

The present invention is distinct from another one in this domain — Roger Yallouz' invention in France No. 1 541 165, patent granted on 26 August 1968, in which a single conduction electrode is applied to the skin and a wire connected therewith is passed round the body or part of the body — in the following respects:

(1)    The present invention invariably makes use of at least two pressure electrodes which are interconnected by an insultated wire.

(2)    In the present invention it is generally sufficient for the wire connecting two adjacent pressure electrodes to run substantially along one side of the body.

It has been found that the wearing of such a garment enables equalization of muscular tone and, therefore, also of the supply of blood to and the temperature in different organs and muscle bundles.

The invention also relates to a garment made according to the aforesaid process, in the manufacture of which means of adjustment to size have been used in order to enclose tightly a human body or part of the body of various sizes. The adjustment may be done with a lace, a cling fastener, a belt with buckle or in some other manner. In a very advantageous embodiment of the invention the garment is wholly or partly made in elastic material.

The locations of the respective pressure electrodes generally correspond with points known in acupuncture. For a description of these points, one may consult "An Outline of Chinese Acupuncture", compiled by the Academy of Traditional Medicine and published by Foreign Languages Press, Peking 1975. The application of the external conductor(s) in particular affects the electropotential relation between these acupuncture points and therewith the mechanical state of stress. It appears that especially an unduly great difference in electric tension between points in the human body corresponds with great differences in tension between various spots (acupuncture points). For details refer to R. Voll: "Topographische Lage der Messpunkte der Elektroakupunktur nach Voll", Uelzen 1976

The garment may also be designed as a combination of two or more of the aforesaid garments. As the need arises, it may also be modelled differently in order to envelop the head, knee, neck, chest or other parts of the body and to have a relaxing, soothing or therapeutic effect through equalization of the electric skin potential.

The conductor may be designed as a single or multiple wire interwoven with the material of the garment or knitted into it. Alternatively, such a wire may be fixed onto, within or through the material of the garment. The conductor may itself be insulated or the insulation may be provided by the material of the garment.

The number of electrodes may vary from two to four, but in certain applications greater numbers of electrodes are also advantageous. These may be interconnected into a network, but it is also possible to build up separate networks (in the case of four or more electrodes). The configuration of the electrodes may be symmetrical relative to the bodily structure, but it may alternatively also be centred on one asymmetrically located part or organ of the body.

Example I

The garment is designed as an abdominal belt 60 cm long, made wholly or partly of woven stretch fabric with a relatively high proportion of elastic and having an ultimate elongation of over 100%. The central part is provided with electrodes spaced apart 10 cm between centres in the longitudinal direction. Each electrode measures 2 cm in the long direction of the belt and 1 cm across. A press stud serves as a fastener. A further electrode measuring 1 x 1 cm has been fitted at 5 cm from both ends of the belt. Each terminal electrode is linked in galvanic contact to the near-by central electrode through a number of interwoven insulated copper wires (the electrodes also being made of copper). The abdominal belt is designed for a girth of 80 – 85 cm. It has been found that wearing of this belt had a beneficial effect on the posture of the wearer such as, for example, in cases of lordosis. Instead of copper another material such as brass or silver may be used. The electrodes can be glued to the fabric, incorporated into the texture or tacked onto the fabric by means of tiny holes in the metal. The conductor may be applied either on/in the stretch fabric or in a non-stretch part of the garment. The belt may also take the form of a waist-belt that can be adjusted by means of a buckle.

0107258

Example II

The garment is a fully continuous band that can be pulled over the elbow. When applied, it shows a tangential elongation of about 50%. There are two electrodes on the inside of the elbow at the point which is known in acupuncture as "large intestine 9, 10, 11" or the point known as "triple heater 11".

Example III

The garment is designed as a long-legged pantie hose. The first electrode is fitted just above the upper end of the perineum on the back side, and the second electrode on the lower point of attachment of the Achilles' tendon of the other leg, whilst the uppermost electrode is connected to the other one(s). The wearing of such a garment often brings relief in cases of excessive fluid being present in tissues, by stimulating the urge to secrete fluid. In like manner a vest, whether or not with sleeves, may be designed as a garment according to the invention. A sleeved vest can often be replaced by a shoulder piece, taking the form of a vest without "body" having one or two arm/shoulder parts.

0107258

CLAIMS

1. A process for the manufacture of a garment that is intended tightly to enclose a human body or a part thereof having a certain size, characterized in that on the side of the garment to be turned to the body at least a first and a second pressure electrode are fitted, which are in galvanic contact through an otherwise insulated flexible conductor of sufficient length to bridge the distance between the interconnected pressure electrodes as measured on the garment when applied to the body.

2. A garment made according to the process of claim 1, characterized in that in its manufacture means of adjustment have been used with a view to tightly enclosing a human body or part of the body of various sizes.

3. A garment made according to the process of claim 1, characterized in that in its manufacture use has been made of elastic material with a view to tightly enclosing a human body or part of the body of various sizes, when the material is stretched to some extent.

4. A garment according to claim 1, 2 or 3, characterized in that it has been designed as a band or belt with a fastening device.

5. A garment according to claim 2 or 3, characterized in that it has been designed as a continuous closed band or belt.

6. A garment according to claim 1, 2 or 3, characterized in that it has been designed as trouser-shaped garment, a vest, a shoulder piece, or as a bodice, that is a vest without neck piece and shoulder straps.

7. A garment according to claim 2,3,4 or 5, characterized in that it has been designed as an abdominal belt, a neck band or a fillet.

6

0107258

8. A garment according to claim 2 or 3, characterized in that it has been designed as a sock, whether or not with heel or toe piece.

9. A garment according to claim 2, 3, 4 or 5, characterized in that it has been designed as a wrist, knee, ankle, instep or elbow band.

10. A garment according to claim 2 or 3, characterized in that it has been designed as a face mask, optionally provided with holes for at least one part of the face.

11. A garment according to any one of claims 2 - 10, characterized in that a third and a fourth pressure electrode have been fitted which are connected by a second conductor so as to form an at least virtually symmetrical configuration relative to the centre face of the elastic band, with the third and the fourth pressure electrode fitted on one longitudinal half and the other two pressure electrodes fitted on the other longitudinal half.

12. A garment according to any one of claims 2 -11, characterized in that the flexible conductor has been designed as at least one insulated wire that has been interwoven with the elastic material.

13. Textile material suitable for the manufacture of a garment according to any one of claims 1 - 12, characterized by the incorporation into the material of two or more pressure electrodes connected together through one or more insulated flexible electrodes.

14. Textile material according to any one of claims 1 - 13, characterized by electrodes substantially made of copper.

0107258

**EUROPEAN SEARCH REPORT**

Application number

EP 83 20 1511

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | FR-A-1 541 165 (YALLOUZ) <br><br> * Page 1, left-hand column, paragraphs 2,4,9 and right-hand column, paragraphs 1-6 and 10,11; figure 6 * <br><br>--- | 1-7,10 ,12-14 | A 61 N 1/14 <br> A 61 N 1/22 |
| A | WO-A-8 202 148 (MONOI) <br><br> * Abstract; figure 1 * <br><br>--- | 1,5,6, 12 | |
| A | WO-A-8 200 951 (WALLANT) <br><br> * Page 8, lines 2-9; page 9; page 16, lines 8-14; figure 16 * <br><br>--- | 2-4,6, 11,12 | |
| A | DE-A-2 840 175 (WIGGENHAUSER) <br> * Page 9; page 13, lines 1-4 * <br><br>--- | 6,13 | TECHNICAL FIELDS SEARCHED (Int. Cl. 3) |
| A | FR-A- 750 222 (WEGNER) <br> * Page 4, lines 32-38; page 2, lines 39-41 * <br><br>--- | 6-9 | A 61 N |
| A | US-A-3 971 387 (MANTELL) <br> * Column 1, line 53 - column 2, line 33 * <br><br>----- | 10 | |

The present search report has been drawn up for all claims

| Place of search <br> THE HAGUE | Date of completion of the search <br> 20-01-1984 | Examiner <br> SIMON J.J.E. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

  & : member of the same patent family, corresponding document

EPO Form 1503. 03.82